# EUROPÄISCHE PATENTSCHRIFT

(11) **EP 4 371 541 B1**
(45) Veröffentlichungstag und Bekanntmachung des Hinweises auf die Patenterteilung: **27.08.2025**
(21) Anmeldenummer: 22020565.2
(22) Anmeldetag: 17.11.2022
(51) Int. Cl.: A61F 13/06

(54) **KOMPRESSIONSBEKLEIDUNGSSET**
COMPRESSION GARMENT SET
ENSEMBLE VÊTEMENT DE COMPRESSION

(43) Veröffentlichungstag der Anmeldung: 22.05.2024
(73) Patentinhaber: medi GmbH & Co. KG, 95448 Bayreuth (DE)
(72) Erfinder: Hoffeins, Peter, 95448 Bayreuth (DE)

(56) Entgegenhaltungen:
- DE-A1- 102013 022 088
- DE-B3- 102019 212 740
- US-A- 5 823 195

## Beschreibung

Die vorliegende Erfindung bezieht sich auf ein Kompressionsbekleidungsset zur Ausübung einer Kompression auf ein Körperteil bestehend aus zumindest einem Kompressionsartikel sowie zumindest einer dem Kompressionsartikel zugehörigen Pelotte nach dem Oberbegriff des Anspruchs 1.

Derartige Artikel zur Ausübung einer Kompression auf ein Körperteil werden insbesondere in Form von Strümpfen, vorzugsweise in Form von Waden- oder Oberschenkelstrümpfen, oder beispielsweise als Strumpfhosen für ein Bein und/ oder Fuß eines Trägers für eine Kompressionstherapie ausgebildet. Auch ist es bekannt Handschuhe und Zehenkappen mit Kompression zu versehen oder bspw. Kompressionsbekleidung anzufertigen. Letztere dienen dazu Kompression auf den Thorax eines Patienten auszuüben. Hierzu weisen die Kompressionsartikel mindestens eine, vorzugsweise mehrere, kompressive Bereiche auf, welche dazu dienen gezielt Druck auf den Körper eines Patienten auszuüben. Der auf den Körper eines Patienten ausgeübte Druck wird hierbei als Kompression bezeichnet. Ziel bei derartigen kompressiven Artikeln ist es, insbesondere bei einem medizinischen Einsatz, ein geschädigtes Venen- und/ oder Lymphsystem eines Patienten zu entlasten. Durch den zugeführten Druck wird eine zunehmende Schwellung der Gliedmaßen vermieden, der Abtransport von venösem Blut und Lymphe verbessert und die Blutzufuhr gesteigert. Bei Einsatz von diesen kompressiven Artikeln in dem Sportbereich bewirken diese eine Leistungssteigerung bzw. eine verbesserte Regeneration.

Zur Herstellung, insbesondere zur Messung und Gütesicherung, von kompressiven Artikeln, insbesondere von gestrickten Arm- oder Beinstrümpfen für die medizinische Anwendung, existiert die RAL-GZ 387 der Gütezeichengemeinschaft. Aus den Prüfbestimmungen der RAL kann entnommen werden, wie bspw. der Druck eines Kompressionsstrumpfes auf ein Bein zu bestimmen ist. Als Messmittel, insbesondere Kompressionsprüfgerät, wird die Prüfung am HOSY Messgerät (Institut Hohenstein) vorgeschlagen.

Neben den zuvor beschriebenen kompressiven Artikeln zur Kompressionstherapie, insbesondere zur Lymph- oder Ödemtherapie, sind auch kompressive Bandagen, bspw. in Form von Sprunggelenk- oder Kniegelenkbandagen, bekannt, die in der Regel aus einem textilen schlauchförmigen und vorzugsweise kompressiven Grundkörper bestehen. Die Kniebandage ist dabei insbesondere als Patellarsehnenbandage ausgebildet.

Die Patellarsehne ist Teil des Kniegelenks und erfüllt eine wichtige Funktion bei der Stabilisierung und Beweglichkeit des Kniegelenks. Sie verbindet über die Kniescheibe den Oberschenkelmuskel Quadrizeps femoris mit dem Schienbein. Über die Patellarsehne, welche eine sehr schmale Sehne ist, wird so die gesamte Krafteinwirkung des Oberschenkelmuskels auf den Unterschenkel übertragen. Insbesondere bei der Beugung und Streckung des Knies kommt es daher zu einer Beanspruchung der Sehne. Bei einer hohen Belastung des Kniegelenks, wie sie zum Beispiel beim Sport vorkommt, oder durch eine Fehlbelastung, kann es zu einer Reizung der Patellarsehne kommen, was sich oftmals als Schmerz im Knie, insbesondere beim Treppensteigen oder Bergabgehen, äußert. Betroffen sind meist Sportler, welche ruckartige bzw. abrupte Bewegungen in ihrer Sportart ausführen.

Nun ist es ferner bekannt die eingangs beschriebenen Kompressionsartikel für eine Kompressionstherapie mit sogenannten Pelotten, insbesondere Druckkörpern, auszustatten. Diese dienen dazu, zusätzlichen Druck auf eine gewünschte Stelle oder einen bestimmten Bereich auszuüben. Ebenso kann durch die Verwendung von solchen Pelotten bei Kompressionsartikeln auch eine gleichmäßige Druckverteilung erzielt werden. Insbesondere eine zirkulär gleichmäßige Druckverteilung ist Voraussetzung für eine effektive Kompressionstherapie. Nach dem Laplace'schen Gesetz ist der Druck proportional zur Spannung des Kompressionsmaterials, aber umgekehrt proportional zum Radius des daruntergelegenen Gewebes. So ist bspw. nach dem Laplace'schen Gesetz der auf einen Knochenvorsprung, wie der Achillessehne oder der Tibiakante, ausgeübte Druck höher als auf eine ebene Fläche. Um einen gleichmäßigen Druck zu erhalten, werden nun durch die Anordnung von den Pelotten, insbesondere in Form von Pads, kleine Radien, wie zum Beispiel rechts und links der Achillessehne, abgepolstert. Dadurch lassen sich Druckschädigungen vermeiden, eine gleichmäßige Druckverteilung erzeugen und zudem die Abheilung verbessern.

Bei Anordnung dieser Pelotten an den kompressiven Grundkörpern der zuvor erwähnten kompressiven Bandagen, dienen sie hier ebenfalls dazu, lokal Druck auf ein Körperteil auszuüben. Bei Ausbildung der Bandage bspw. als Kniebandage, dient diese dazu gezielt Druck auf die Patellarsehne auszuüben. Durch diesen Druck wird der Schmerz am Knie vermieden, zumindest deutlich verringert.

Aus dem Stand der Technik sind eine Vielzahl von Kompressionsartikeln zur Ausübung einer Kompression auf ein Körperteil bekannt, die insbesondere zusätzlich eine Pelotte umfassen.

Ein derartiger Kompressionsartikel, insbesondere in Form eines kompressiven Strumpfes, ist beispielsweise aus der EP 1 391 190 B1 bekannt.

Der Kompressionsstrumpf ist hierbei aus einem elastischen Gewebe, Gewirke oder Gestrick ausgebildet. Im Bereich eines Kniegelenkes ist zwischen dem Kompressionsstrumpf und dem Kniegelenk ein als Pelotte ausgebildeter Kompressionskörper, welcher aus einem elastischen Material und einer Aussparung zur Aufnahme einer Kniescheibe eines Kniegelenks aufweist, angeordnet. Dieser ist an der Innenseite des Kompressionsstrumpfes fest angebracht.

Ein weiterer Kompressionsartikel, ausgebildet als Sprunggelenkbandage, ist beispielsweise aus der EP 2 594 233 B1.

Die orthopädische Bandagenanordnung für ein Sprunggelenk umfasst einen sockenartigen Gestrickgrundkörper und ein mit dem Gestrickteil verbindbares Gurtteil. Das Gurtteil weist einen länglichen Gurtgrundkörper auf, wobei von diesem wenigstens ein Gurtabschnitt abragt, welcher im getragenen Zustand der Bandagenanordnung sich an der Außenseite des Fußes erstreckt. Der Gestrickgrundkörper kann nun wenigstens eine sich im getragenen Zustand über das Sprunggelenk erstreckende Pelotte aufweisen. Die Pelotte erhöht hierbei die orthopädisch wirksame Funktion des Gestrickgrundkörpers bzw. des Gestrickteils und somit der Bandagenanordnung insgesamt. Die Pelotte ist hierbei ebenfalls in den Gestrickgrundkörper der Bandagenanordnung eingearbeitet.

Auch die DE 20 2014 011 197 U1 offenbart einen Kompressionsartikel, insbesondere eine Kniegelenkbandage und ein Pelottensystem dazu.

Die Kniegelenkbandage umfasst hierbei einen Gestrickkörper. Vorderseitig ist ein erster Spanngurt vorgesehen, der nur abschnittsweise um den Gestrickkörper umläuft und sich über zumindest eine Pelotte erstreckt. Rückseitig und höhenmäßig versetzt zum ersten Spanngurt ist ein zweiter Spanngurt vorgesehen, der ebenfalls nur abschnittsweise um den Gestrickkörper umläuft. Beide Gurte sind zur Einstellung eines Drucks auf die Pelotte ausgebildet, wobei der erste Spanngurt sich direkt über die Pelotte erstreckt. Bevorzugt sind mehrere Pelotten vorgesehen. Die Pelotten sind hierbei in Taschen aufgenommen, die am Gestrickkörper angebracht sind, wobei die Taschen partiell offen sein können, so dass die Pelotten gegebenenfalls entnommen und bei Bedarf gegen vom Material her etwas härter oder weicher ausgelegte Pelotten ausgetauscht werden können. Alternativ können die Taschen auch allseits geschlossen sein, so dass eine Entnahme oder ein Austausch der Pelotten nicht möglich ist.

Eine Pelotte, insbesondere einer Knöchelpelotte, in Kombination mit einem Kompressionsartikel ist schließlich auch aus der US 5,823,195 A bekannt. Als Kompressionsartikel wird dabei vorgeschlagen einen Kompressionsstrumpf, vorzugsweise mit einem graduierten Druckverlauf, zu verwenden. Die Pelotte wird dabei durch den Kompressionsartikel, insbesondere durch den Kompressionsstrumpf oder einer Bandagierung, an dem Bein eines Trägers fixiert. Alternativ kann die Pelotte auch mit Hilfe eines Klebebandes an dem Bein befestigt werden. Dann ist es möglich die Pelotte auch mit einem gewöhnlichen Strumpf, also ohne Kompression, zu tragen.

Als nachteilig dieser Ausgestaltung der Kompressionsartikel sowie der zugehörigen Pelotten erweist sich, dass die Pelotten, egal an welchen Positionen und zu welchen orthopädischen Zwecken, diese in oder an dem Kompressionsartikel befestigt sind. Die Pelotten sind hierbei in den Kompressionsartikeln, insbesondere in deren Gestrickgrundkörper, integriert, so dass sich eine lagefeste Verbindung der Pelotte mit dem Kompressionsartikel ergibt.

Diese Verbindung der Pelotte mit dem Kompressionsartikel hat einen erhöhten Prozess- und Verarbeitungsaufwand zur Folge. Wie im Stand der Technik beschrieben, werden die Pelotten üblicherweise in an dem Kompressionsartikel angebrachten Taschen aufgenommen. Zur Ausbildung dieser Taschen sind neben den Materialkosten, insbesondere die Herstellkosten, insbesondere das Aufkleben, Aufschweißen oder Annähen des Taschenmaterials an den Kompressionsartikeln zu berücksichtigen. Zudem muss die Pelotte noch manuell in die Tasche eingebracht werden, was ebenfalls Kostenaufwand verursacht.

Als nachteilig erweist sich ebenfalls, dass wenn die Pelotte fest mit dem Kompressionsartikel verbunden ist, der Kompressionsartikel immer nur gemeinsam mit der Pelotte getragen werden kann. Ein Auf- oder Abrüsten des Kompressionsartikels, also die Verwendung des Kompressionsartikels mit oder ohne Pelotte, abhängig vom Verlauf der Kompressionstherapie ist nicht möglich. So muss bspw. bei einem positiven Therapieverlauf, wonach kein zusätzlicher Druckkörper benötigt wird, ein neuer Kompressionsartikel angeschafft werden. Dies ist mit zusätzlichen Kosten für den Patienten verbunden.

Zudem ergibt sich dadurch auch der Nachteil, dass die Lage, also die Position der Pelotte an dem Kompressionsartikel, unveränderbar, also fest bestimmt, ist. D. h. eine Passform des Kompressionsartikels ist nach der Fertigung nicht mehr optimierbar bzw. korrigierbar. Wurde bspw. die Lage der Pelotte an dem Kompressionsartikel vor der Fertigung des Kompressionsartikels falsch bestimmt, bspw. durch ungenaue Bestellangaben, kommt es zu Fertigungsfehler oder Fertigungstoleranzen, bspw. bei einem Kompressionsstrumpf, so kann dieser durch den Anwender nicht getragen werden. Der kompressive Strumpf müsste erneut angefertigt werden, was zusätzliche Kosten für den Patienten oder bei einer Reklamation für den Hersteller des Kompressionsartikels zur Folge hat. Die durch die Pelotte lokal erhöhte Kompressionswirkung würde nämlich dann an einer falschen Körperstelle eines Patienten ausgeübt werden.

Einen weiteren Nachteil, welcher auch dann besteht, selbst wenn die Pelotte lösbar von dem Kompressionsartikel, insbesondere herausnehmbar aus einer Pelottentasche ausgebildet ist, ist dass die Pelottenform bzw. Geometrie durch die Tasche vorgegeben ist. Dadurch ist es nicht möglich, genauso wie bei einer an dem Kompressionartikel fest angeordneten Pelotte, zwischen mehreren Pelottenformen zu variieren, dies insbesondere während der Kompressionstherapie.

Aber auch unabhängig von der Art und Weise, wie die Pelotten an den Kompressionsartikeln befestigt sind, also lösbar oder unlösbar, bringt alleine die Tatsache, dass die Pelotten an den Kompressionsartikeln angeordnet sind, den Nachteil mit sich, dass dadurch die An- und Auszziehbarkeit der Kompressionsartikel weiter erschwert wird. Es ist allgemein bekannt, dass kompressive Artikel, insbesondere kompressive Strümpfe oder Strumpfhosen, an sich schon schwer anzuziehen sind. Die Anordnung von weiteren Komponenten, insbesondere einer oder mehrerer Pelotten, erschwert die An- und Ausziehbarkeit, auf Grund ihrer Formen und Materialien, zusätzlich. Die Pelotten sind nämlich meist aus einem starren Material gefertigt. Der Tragekomfort, welcher auch die An- und Ausziehbarkeit des Kompressionsartikels umfasst, verschlechtert sich dadurch jedenfalls.

Der vorliegenden Erfindung liegt daher die Aufgabe zugrunde ein Kompressionsbekleidungsset bereit zu stellen, welches die Nachteile aus dem Stand der Technik vermeidet, insbesondere die Passgenauigkeit und den Tragekomfort des Kompressionsbekleidungssets deutlich verbessert.

Gemäß der Erfindung besteht das erfindungsgemäße Kompressionsbekleidungsset zur Ausübung einer Kompression auf ein Körperteil aus zumindest einem Kompressionsartikel sowie zumindest einer dem Kompressionsartikel zugehörigen Pelotte, wobei die Pelotte in der Tragestellung des Kompressionsbekleidungssets unter dem Kompressionsartikel zum Liegen kommt und wobei die Pelotte an einer in der Tragstellung zum Träger gerichteten Seite zumindest eine Haftfläche aufweist, so dass die Pelotte losgelöst, also unabhängig, von dem Kompressionsartikel an der Haut eines Trägers positionierbar bzw. auch dort befestigbar ist. D.h. die Pelotte weist eine haftende, bevorzugt leicht klebrige, adhäsive, jedenfalls selbsthaftende Oberfläche auf. Diese ist insbesondere derart ausgebildet, dass die Pelotte selbst, also ohne zusätzliche Hilfe, wie zum Beispiel einem Stützgurt, welcher bspw. die Pelotte und das Körperteil des Trägers allseitig umgibt, an der Haut eines Trägers haftet. Zudem ist die Haftfläche bevorzugt derart ausgebildet, dass die Pelotte wieder lösbar von der Haut eines Trägers ist. D.h. wiederum, dass keine externen Mittel, wie zum Beispiel Lösemittel, benötigt werden, um die Pelotte wieder von der Haut eines Trägers zu entfernen. Besonders bevorzugt ist die Pelotte, insbesondere die Haftfläche, derart ausgebildet, dass diese mehrfach verwendet werden kann. Es handelt sich somit bevorzugt um eine wiederverwendbare Pelotte.

Die zumindest eine Pelotte derart ausgebildet, dass diese an einer in der Tragestellung zum Träger abgewandten Seite zumindest ein Trägerelement für zumindest ein daran angebrachtes Pelottenelement aufweist. Das zumindest eine Trägerelement ist hierbei bevorzugt aus einer sehr dünnen und reißfesten Trägerfolie mit einer friktionsarmen Oberfläche an der Außenseite gefertigt, so dass die Pelotte, an der dem Kompressionsartikel zugewandten Seite erhöhte Gleiteigenschaften aufweist. Das Anziehen des Kompressionsartikels wird durch die friktionsarme Oberfläche an der Außenseite der Pelotte deutlich erleichtert. Die Pelotte erfüllt somit zusätzlich den Zweck ähnlich einer Anziehhilfe für Kompressionsstrümpfe oder Bandagen.

Gemäß der Erfindung besteht die zumindest eine Pelotte oder das zumindest eine Pelottenelement aus einem flexiblen bzw. elastischen, plastisch verformbaren Kunststoffmaterial. Das Kunststoffmaterial kann hierbei unterschiedliche Materialeigenschaften, insbesondere Härtegrade, aufweisen. Besonders bevorzugt besteht das zumindest eine Pelottenelement aus einem Silikon mit einer adhäsiven Oberfläche, so dass das Pelottenelement selbst die zumindest eine Haftfläche bildet. Alternativ kann die zumindest eine Haftfläche durch eine an der Pelotte oder dem zumindest einen Pelottenelement aufgebrachte Klebeschicht gebildet sein. Die Klebeschicht kann hierbei vollflächig oder nur lokal an einer oder mehreren Stellen der Pelotte aufgebacht sein.

Gemäß einem weiteren Ausführungsbeispiel weist die Pelotte oder das zumindest eine Pelottenelement auf der in der Tragstellung zum Träger gerichteten Seite eine an die Anatomie des Körperteils in der Trageposition angepasst Geometrie auf. Dabei ist die Pelotte oder das zumindest eine Pelottenelement auf dieser Seite im Wesentlichen eben, in einer oder mehrerer Richtungen konvex oder konkav gekrümmt, ausgebildet. D.h. die Pelotte weist, auf der dem Träger zugewandten Seite bevorzugt nur sehr wenige, bevorzugt keine Vor- oder Rücksprünge auf. Hierdurch kann die Plotte vollflächig an der Haut eines Trägers positioniert werden, also mit einer größtmöglichen Fläche. Hierdurch wird ein bestmöglicher Halt der Pelotte an der Haut eines Trägers erzielt.

Alternativ weist die Pelotte oder das zumindest ein Pelottenelement auf der in der Tragstellung zum Träger gerichteten Seite zumindest einen Rücksprung auf oder es können auch mehrere aneinander anschließende oder voneinander beabstandete Pelottenelemente an dem Trägerelement angebracht sein. Ziel hierbei ist es in der Tragestellung unterhalb der Pelotte zumindest einen, bevorzugt mehrere, Transportkanäle für Lymphflüssigkeit auszubilden. Dadurch kann die Pelotte zudem auch an Köperstellen mit engen Radien angebracht werden, da durch den zumindest einen Rücksprung oder durch die beanstandeten Pelottenelemente die Pelotte, insbesondere dann wenn sie aus einem flexiblen Material gefertigt ist, entsprechend der vorgegebenen Anatomie verformbar ist.

Besonders bevorzugt ist die zumindest eine Pelotte an einer in der Tragestellung zum Träger abgewandten Seite im Wesentlichen konvex gekrümmt. Die konvexe Krümmung der Pelotte unterstützt die gleichmäßige Druckverteilung des darüberliegenden und am Körperteil angeordneten Kompressionsartikels.

Gemäß einem weiteren Ausführungsbeispiel weist die Pelotte eine oder mehrere Schneidemarkierungen auf, so dass die Pelotte in ihrer Form individuell anpassbar ist. Dabei kann es sich bspw. bevorzugt um eine oder mehrere auf die Pelotte aufgedruckte Markierungen, insbesondere Linien, handeln. Alternativ können diese Markierungen auch als Perforationen im Material der Pelotte ausgebildet sein. Hierdurch kann die Größe Pelotte individuell an das Körperteil des Trägers angepasst werden. Dies mit oder ohne einem externen Werkzeug. Zudem können die Schneidemarkierungen des Weiteren Kennzeichen umfassen, die auf eine Größe der Pelotte oder des Körperteils Bezug nehmen, bspw. Größenangaben wie S bis XL, oder Umfangsmaße, bspw. in der Maßeinheit Millimeter oder Zentimeter.

Gemäß einem weiteren Ausführungsbeispiel weist das Kompressionsbekleidungsset ferner zumindest ein lösbar an der zumindest einen Haftfläche der Pelotte befestigbares Abdeckelement auf. Dadurch wird die Haftfläche der Pelotte in einem freiliegenden Zustand vor Verunreinigungen geschützt. Das lösbare Abdeckelement ist hierbei bevorzugt als Kunststofffolie ausgebildet, welche die gesamte Haftfläche bedeckt. Die Pelotte wird bevorzugt mit dem Abdeckelement ausgeliefert. Vor der Benutzung wird diese durch den Anwender entfernt und anschließend, also nach der Anwendung, wieder an der Haftfläche angebracht. Bevorzugt umfasst das Set ferner ein Mittel zur Reinigung der Haftfläche, so dass die zumindest eine Pelotte wiederverwendbar ist. Das Mittel ist dabei bevorzugt derart ausgebildet, dass es Öl- und Fettverschmutzungen, Staub, Schmutz, Make-Up, Hautschuppen, Hautpartikel und Haare von der Haftschicht löst, ohne die Haftschicht dabei zu beschädigen.

Besonders bevorzugt ist der Kompressionsartikel ein kompressiver Strumpf, insbesondere ein Arm- oder Beinstumpf, mit oder ohne Hand- oder Fußteil, eine kompressive Socke, eine Zehenkappe, ein kompressiver Handschuh, eine Bandage, ein einstellbares, insbesondere adaptives Kompressionstextil, wie zum Beispiel ein sogenannter Compression Wrap, eine kompressive Weste, oder ein kompressives Element einer Orthese.

Bevorzugt betragen die durch den Kompressionsartikel erzeugten kompressiven Druckwerte zwischen 5 und 60 mmHg, bevorzugt zwischen 10 und 45 mmHg, besonders bevorzugt zwischen 15 und 25 mmHg. Die genannten Kompressionswerte können, insbesondere für die kompressiven Arm- und Beinstrümpfe, durch die eingangs beschriebene Messanleitung und Messmethodik, insbesondere nach der RAL-GZ 387 der Gütezeichengemeinschaft sowie am HOSY Messgerät (Institut Hohenstein) ermittelt werden.

Das vorliegende Kompressionsbekleidungsset zeichnet sich durch eine Reihe erheblicher Vorteile aus.

Durch die Ausbildung des Kompressionsbekleidungsset derart, dass die Pelotte losgelöst, also unabhängig, von dem Kompressionsartikel an der Haut eines Trägers positionierbar und dort befestigbar ist, insbesondere durch deren Haftfläche, ist es möglich, dass die Pelotte exakt auf der zuvor festgelegten und vorzugsweise durch ein Fachpersonal bestimmten lokalen Stelle am Körperteil eines Trägers platziert werden kann. Es wird dadurch bezüglich der Anordnung und Ausrichtung der Pelotte an dem Körperteil eine hundertprozentige Passgenauigkeit erzielt. Reklamationen diesbezüglich, also auf Grund falsch in ein Kompressionsartikel eingearbeiteter Pelotten, wie sie aus dem Stand der Technik bekannt sind, so dass die Pelotten unkorrekt am Körperteil eines Patienten zum Liegen kommen, können gänzlich ausgeschlossen werden.

Zudem ist es durch die erfindungsgemäße Ausbildung des Kompressionsbekleidungssets möglich, dieses, insbesondere den Kompressionsartikel, individuell an den Verlauf der Kompressionstherapie anzupassen. So kann das Kompressionsbekleidungsset auf- oder abgerüstet werden. Bspw. ist es möglich, den Kompressionsartikel mit oder ohne Pelotte zu verwenden. Auch kann bspw. aus einer Vielzahl von Pelotten mit unterschiedlichen Eigenschaften, insbesondere hinsichtlich Materials, Hafteigenschaft und/ oder Form, abhängig von einer Passgenauigkeit und/ oder einem Therapieverlauf ausgewählt werden. Es ist somit nicht mehr nötig neben der Pelotte zusätzlich auch noch den Kompressionsartikel auszutauschen, wie es aus dem Stand der Technik bekannt ist.

Einen weiteren Vorteil der Erfindung stellt die deutlich leichtere An- und Ausziehbarkeit des Kompressionsbekleidungssets dar. Dadurch wird der Tragekomfort wesentlich erhöht. Zu diesem gehört nämlich auch der Schwierigkeitsgrad der An- und Ausziehbarkeit des Kompressionsartikels. Die von Grund auf meist nur mit körperlichem Aufwand an- und ausziehbaren Kompressionsartikel, bspw. kompressiven Strümpfe, werden nun gemäß der Erfindung nicht mehr zusätzlich mit weiteren Komponenten, also mit einer oder mehrerer Pelotten, versehen, welche die An- und Ausziehbarkeit zusätzlich erschweren. Die Pelotte ist nämlich erfindungsgemäß von dem Kompressionsartikel entkoppelt. Es ist somit nicht mehr notwendig den Kompressionsartikel gleichzeitig mit einer Pelotte anzuziehen.

Auch einen wesentlichen Vorteil stellt die Möglichkeit dar, einen geschlossenen Kompressionsartikel, bspw. einen rundgestrickten Kompressionsstrumpf oder nahtlos gefertigten Kompressionsstrumpf auf einer Flachstrickmaschine für eine derartige Kompressionstherapie mit Pelotte zu verwenden. Diese Artikel fanden bisher für die zuvor genannten Therapie keine Berücksichtigung. Grund hierfür ist, dass es nur mit erheblichem Aufwand oder teilweise gar nicht möglich ist Pelotten in diese in Umfangsrichtung geschlossen Artikel einzuarbeiten.

Schlussendlich ist auch noch festzuhalten, dass das erfindungsgemäße Kompressionsbekleidungsset einen deutlichen Kostenvorteil gegenüber der bisherigen am Markt erhältlichen kompressiven Artikel mit sich bringt. Es ist nicht mehr notwendig an den Kompressionsartikeln Vorkehrungen zu treffen, um die Pelotte daran zu befestigen. D.h. es müssen keine Taschen an den Artikeln mehr ausgebildet werden. Die Pelotten müssen bei der Fertigung des Artikels auch nicht mehr manuell in der Tasche platziert werden. Dadurch werden die Herstellkosten deutlich reduziert.

Nachfolgend wird die Erfindung anhand mehrerer Ausführungsbeispiele und in Verbindung mit den beigefügten Zeichnungen erläutert.

Dabei zeigen:
Figuren 1A bis 1C ein erstes Ausführungsbeispiel des erfindungsgemäßen Kompressionsbekleidungssets bestehend aus einem kompressiven Beinstrumpf und einer Knöchelpelotte in einer dreidimensionalen Darstellung, nämlich in einem ungetragenen, teilweise getragenen und getragenen Zustand,
Figuren 2A bis 2C ein zweites Ausführungsbeispiel des erfindungsgemäßen Kompressionsbekleidungssets bestehend aus einer kompressiven Zehenkappe und einer Fußpelotte ebenfalls in einer dreidimensionalen Darstellung, nämlich in einem ungetragenen, teilweise getragenen und getragenen Zustand,
Figuren 3A bis 3C ein drittes Ausführungsbeispiel des erfindungsgemäßen Kompressionsbekleidungssets bestehend aus einem kompressiven und einstellbaren Kompressionstextil und einer Fußpelotte in einer dreidimensionalen Darstellung, nämlich in einem ungetragenen, teilweise getragenen und getragenen Zustand,
Figuren 4A bis 4C ein viertes Ausführungsbeispiel des erfindungsgemäßen Kompressionsbekleidungssets bestehend aus einer kompressiven Bandage und einer Kniepelotte in einer dreidimensionalen Darstellung, nämlich in einem ungetragenen, teilweise getragenen und getragenen Zustand,
Figuren 5A bis 5C ein fünftes Ausführungsbeispiel des erfindungsgemäßen Kompressionsbekleidungssets bestehend aus einem kompressiven Handschuh und einer Handpelotte in einer dreidimensionalen Darstellung, nämlich in einem ungetragenen, teilweise getragenen und getragenen Zustand,
Figuren 6A bis 6C ein sechstes Ausführungsbeispiel des erfindungsgemäßen Kompressionsbekleidungssets bestehend aus einer kompressiven Weste und einer Thoraxpelotte in einer dreidimensionalen Darstellung, nämlich in einem ungetragenen, teilweise getragenen und getragenen Zustand,
Figuren 7A bis 7C die Knöchelpelotte gemäß dem ersten Ausführungsbeispiel des erfindungsgemäßen Kompressionsbekleidungssets umfassenden den kompressiven Beinstrumpf aus den Figuren 1A bis 1C in einer dreidimensionalen Darstellung und in einer Schnittdarstellung,
Figuren 8A bis 8C die Handpelotte gemäß dem fünften Ausführungsbeispiel des erfindungsgemäßen Kompressionsbekleidungssets umfassenden den kompressiven Handschuh aus den Figuren 5A bis 5C in einer dreidimensionalen Darstellung und in einer Schnittdarstellung;

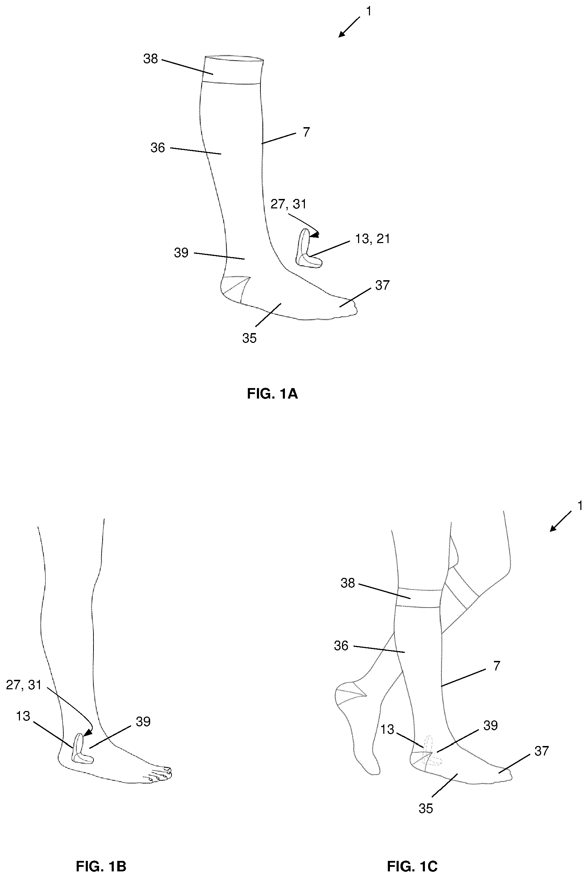

In den Figuren 1A bis 1C ist ein Kompressionsbekleidungsset 1 mit einem Kompressionsartikel in Form eines kompressiven Beinstrumpfes 7 sowie einer Knöchelpelotte 13 gezeigt. Dabei zeigt Figur 1A das Set 1 in einem ungetragenen Zustand. In Figur 1B ist die Knöchelpelotte 13 an dem Fuß eines Trägers positioniert und dort befestigt. Schließlich zeigt Figur 1C den Beinstrumpf 7 und die Pelotte 13 in dem getragenen Zustand. Die Figuren 1A bis 1C veranschaulichen somit auch bildlich das schrittweise Anlegen des Kompressionsbekleidungssets 1.

Wie in der Figur 1A zu sehen ist, ist der Kompressionsartikel, also der Beinstrumpf 7, des Kompressionsbekleidungssets 1, als kompressiver Wadenstrumpf ausgebildet. Dieser umfasst neben einem Fußteil 35 ein vorzugsweise mit einem graduellen Druckverlauf versehenes Wadenteil 36. Der kompressive Druck nimmt hierbei vorzugsweise von distal nach proximal ab. Das Fußteil 35 weist hierbei einen geschlossenen Zehenbereich 37 sowie einen den Knöchel umfassenden Bereich 39 auf. Es ist auch möglich, dass das Fußteil 35 mit einem offenen Zehenbereich ausgebildet ist. Neben einem ersten Abschnitt, vorzugsweise einer eingestrickten Ferse im Fußteil 35, weist der Wadenstrumpf 7 an dessen oberen Ende einen Bundabschnitt 38 auf, welcher bevorzugt als doppellagiger Bund ausgebildet ist. Der Stumpf 7 besteht hierbei bevorzugt aus einem rund- oder flachgestrickten kompressiven Gestrickteil.

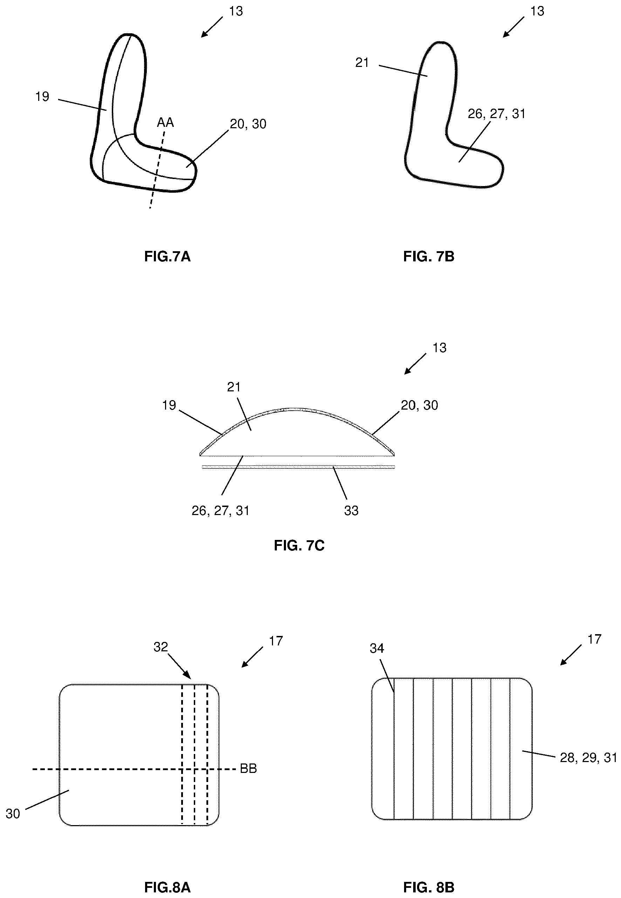

Neben dem kompressiven Wadenstrumpf 7 umfasst nun das Kompressionsbekleidungsset 1 eine Knöchelpelotte 13. Die vorzugsweise L-förmig ausgebildete Pelotte 13 für einen Knöchelbereich 39 weist nun erfindungsgemäß an der in der Tragstellung zum Träger gerichteten Seite 31 eine Haftfläche 27 auf. Hierdurch kann die Pelotte 13, wie folgend in Figur 1A zu sehen ist, losgelöst, also unabhängig, von dem Wadenstrumpf 7 an der Haut eines Trägers positioniert und dort lösbar befestigt werden. Die Knöchelpelotte 13 weist hierzu, wie später in den Figuren 7A bis 7C detailliert beschrieben wird, an einer in der Tragestellung zum Träger abgewandten Seite ein Trägerelement für zumindest ein daran angebrachtes Pelottenelement 21 auf. Das Pelottenelement 21 ist bevorzugt aus einem elastischen oder plastisch verformbaren Kunststoffmaterial gefertigt. Besonders bevorzugt besteht dieses aus einem Silikon mit einer adhäsiven Oberfläche, so dass das Pelottenelement 21 selbst die Haftfläche 27 der Pelotte 13 bildet. Die Knöchelpelotte 13, insbesondere das Pelottenelement 21, weist ferner auf der in der Tragstellung zum Träger gerichteten Seite 31 eine an die Anatomie des Körperteils angepasst Geometrie auf. Da der den Knöchel eines Trägers umgebene Bereich 39 in der Regel im Wesentlichen eben oder leicht konkav gekrümmt ist, ist die Seite 31 der Pelotte 13 bevorzugt ebenfalls eben oder leicht konvex gekrümmt ausgebildet. Alternativ kann die Seite 31 auch im Wesentlichen konkav gekrümmt ausgebildet sein. Die Außenseite der Pelotte 13, also die in der Tragestellung zum Träger abgewandten Seite, ist hierbei bevorzugt konvex gekrümmt ausgebildet.

Die Figur 1B zeigt nun einen Tragezustand des Kompressionsbekleidungssets 1, in dem nur die Knöchelpelotte 13 an dem Fuß eines Trägers, insbesondere in einem Knöchelbereich 39, positioniert ist. Die an der in der Tragstellung zum Träger gerichteten Seite 31 der Pelotte 13 angebrachte Haftfläche 27 ist hierbei derart ausgebildet, dass die Pelotte 13, losgelöst, also unabhängig, von dem Wadenstrumpf 7 an der Haut eines Trägers positioniert bzw. befestigt werden kann. Hierzu ist die Haftfläche 27 als bevorzugt leicht klebrige, adhäsive, jedenfalls selbsthaftende Oberfläche ausgebildet. Somit haftet die Pelotte 13 selbst, also ohne zusätzliche Hilfe, an der Haut des Trägers. Natürlich ist die Hafteigenschaft der Haftfläche 27 derart ausgebildet, dass die Knöchelpelotte 13 wieder lösbar von der Haut eines Trägers ist. Durch diese erfindungsgemäße Ausbildung des Kompressionsbekleidungssets 1, kann die Knöchelpelotte 13 exakt am Körperteil eines Trägers platziert werden.

In Figur 1C ist nun das Kompressionsbekleidungsset 1 in einem Tragezustand gezeigt, d.h. zusätzlich zu der positionierten und angelegten Knöchelpelotte 13 ist nun auch der Beinstrumpf 7 mit dem Fußteil 35, dem Bund 38 und dem Zehenbereich 37 an dem Bein des Trägers angelegt. Dabei wurde, nachdem die Knöchelpelotte 13 an dem Fuß des Trägers positioniert wurde, siehe Figur 1B, der Wadenstrumpf 7 mit dem Wadenteil 36 und dem Fußteil 35 über das Bein bzw. den Fuß des Trägers und schließlich auch über die zuvor im Knöchelbereich 39 angeordnete Pelotte 13 gezogen. Durch diese erfindungsgemäße Ausbildung des Kompressionsbekleidungssets 1, wird die An- und Ausziehbarkeit des Kompressionsbekleidungssets 1 wesentlich verbessert.

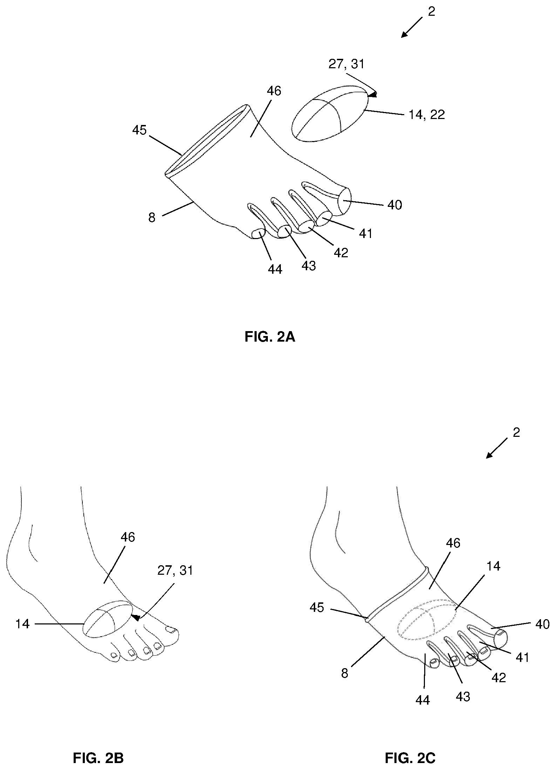

Die Figuren 2A bis 2C zeigen nun ein Kompressionsbekleidungsset 2 mit einem Kompressionsartikel in Form einer kompressiven Zehenkappe 8 sowie einer Fußpelotte 14. Figur 2A zeigt das Set 1 ebenfalls in einem ungetragenen Zustand. Figur 2B zeigt die an dem Fuß eines Trägers positionierte Fußpelotte 14. In Figur 2C ist nun neben der Fußpelotte 14 auch die kompressive Zehenkappe 8 an dem Fuß eines Trägers angelegt. Die Figuren 2A bis 2C veranschaulichen somit ebenfalls das schrittweise Anlegen des Kompressionsbekleidungssets 2.

In Figur 2A ist der Kompressionsartikel des Kompressionsbekleidungssets 2 als kompressive Zehenkappe 8 ausgebildet. Diese weist neben einem Spannbereich 46 und einem Abschlussrand 46 mehrere an dem Spannbereich 46 angebrachte Zehenabschnitte 40 bis 44 auf. Die kompressive Zehenkappe 8 dient dazu, Druck auf den Vorfußbereich auszuüben. Auch bei diesem Ausführungsbeispiel ist der Kompressionsartikel, also die Zehenkappe 8, bevorzugt aus einem rund- oder flachgestrickten kompressiven Gestrickteil gefertigt.

Das Kompressionsbekleidungsset 2 umfasst nun ferner die Fußpelotte 14. Auch die bevorzugt pad-förmig ausgebildete Pelotte 14 für den Spannbereich 46 weist nun erfindungsgemäß an der in der Tragstellung zum Träger gerichteten Seite 31 eine Haftfläche 27 auf. Die Pelotte 14 ist dadurch losgelöst, also unabhängig, von der Zehenkappe 8 an der Haut eines Trägers positionierbar. Die Pelotte 14 weist hierzu ebenfalls, wie auch die Pelotte 13 gemäß Figur 1A, an einer in der Tragestellung zum Träger abgewandten Seite ein Trägerelement für zumindest ein daran angebrachtes Pelottenelement 22 auf. Das Pelottenelement 22 ist hierbei ebenfalls bevorzugt aus einem Silikon mit einer adhäsiven Oberfläche ausgebildet, so dass das Pelottenelement 22 selbst die Haftfläche 27 bildet. Die Fußpelotte 14, insbesondere das Pelottenelement 23, weist gemäß diesem Ausführungsbeispiel auf der in der Tragstellung zum Träger gerichteten Seite 31 eine an die Anatomie des Körperteils angepasst Geometrie auf, insbesondere ist diese bevorzugt eben oder leicht konkav gekrümmt ausgebildet. Die Außenseite der Pelotte 14 hingegen ist hierbei bevorzugt konvex gekrümmt ausgebildet.

Figur 2B zeigt nun einen ersten Tragezustand des Kompressionsbekleidungssets 2. In diesem ist nur die Fußpelotte 14 an dem Fuß, insbesondere an dem Spannbereich 46, eines Trägers positioniert und dort fest angebracht. Dazu wird die Pelotte 14, welche an der in der Tragstellung zum Träger gerichteten Seite 31 die Haftfläche 27 aufweist, im Bereich des Spanns 46 an diesem positioniert bzw. dort befestigt. Hierzu ist die Haftfläche 27 ebenfalls als bevorzugt leicht klebrige, adhäsive, jedenfalls selbsthaftende Oberfläche ausgebildet, so dass die Pelotte 14 ohne externe Hilfe an der Haut des Trägers haftet, also dort befestigt ist. Durch diese erfindungsgemäße Ausbildung des Kompressionsbekleidungssets 2, kann auch die Fußpelotte 14 exakt am Spannbereich 46 des Trägers positioniert und dort befestigt werden.

Schließlich zeigt Figur 2C das Kompressionsbekleidungsset 2 in einem zweiten Tragezustand, nämlich dann, wenn beide Komponenten des Sets 2, also der Kompressionsartikel 8 sowie die Pelotte 14, an dem Fuß des Trägers angelegt sind. Hierzu wurde, nachdem die Fußpelotte 14 an dem Fuß des Trägers positioniert und dort befestigt wurde, die kompressive Zehenkappe 8 mit dem Abschlussrand 45 und den mehreren Zehenabschnitten 40 bis 44 über den Fuß und über die dort zuvor befestigte Pelotte 14 gezogen. Es ist somit nicht mehr notwendig einen Kompressionsartikel mit einer daran befestigten Pelotte, wie es aus dem Stand der Technik bekannt und üblich ist, gemeinsam entlang des Fußes, im konkreten Anwendungsfall über einen Zehenbereich bis in eine Trageposition, nämlich bis die Pelotte 14 in einem Spannbereich angeordnet ist, zu ziehen. Die An- und Ausziehbarkeit des erfindungsgemäßen Kompressionsbekleidungssets 2 wird somit gegenüber den bereits bekannten Lösungen deutlich erleichtert.

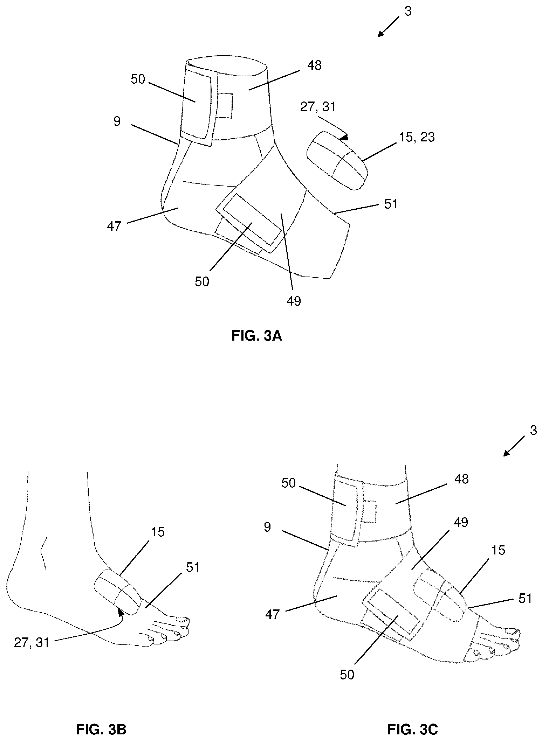

In den Figuren 3A bis 3C ist nun ein Kompressionsbekleidungsset 3 mit einem Kompressionsartikel, ausgebildet als einstellbares bzw. adaptives Kompressionstextil 9, mit einer Fußpelotte 15 gezeigt. Dabei zeigt Figur 3A das Set 3 erneut in einem ungetragenen Zustand. In Figur 3B ist die Fußpelotte 15 an dem Träger positioniert. Gemäß Figur 3C ist das Kompressionsbekleidungsset 3 nun komplett am Fuß des Trägers angelegt.

Wie in Figur 3A zu sehen ist, ist der Kompressionsartikel des Kompressionsbekleidungssets 3 ein einstellbares bzw. adaptives Kompressionstextil 9, insbesondere ein sogenannter Compression Wrap, welcher aus einem Grundkörper 47, an welchem mehrere, bevorzugt zwei, Bänder 48, 49 angeordnet sind, besteht. Die Bänder 48, 49 weisen an ihren Enden jeweils einen Verschluss, vorzugsweise jeweils eine Klettfläche 50 auf, so dass die Bänder 48, 49, nachdem sie um das Körperteil des Trägers, insbesondere um den Fuß und dessen Spannbereich 51 gewickelt wurden, auf sich selbst oder an dem Grundkörper 47 befestigbar sind. Das einstellbare bzw. adaptive Kompressionstextil 9 besteht hierbei bevorzugt aus einem im Wesentlichen unelastischen Gewebe, insbesondere Gewirke. Dieses ist bevorzugt mehrschichtig aufgebaut und zuschneidbar, so dass das Kompressionstextil 9 individuell an das Körperteil des Trägers anpassbar ist.

Nun umfasst das Kompressionsbekleidungsset 3 ebenfalls eine Pelotte 15 mit einer Haftfläche 27 an der in der Tragstellung zum Träger gerichteten Seite 31. Hierdurch kann die Pelotte 15, wie in Ausführungsbeispielen zuvor, losgelöst, also unabhängig, von dem Kompressionsartikel, insbesondere dem Kompressionstextil 9, an der Haut eines Trägers, insbesondere in einem Spannbereich 51 positioniert und dort befestigt werden. Die Fußpelotte 15 ist hierzu bevorzugt ebenfalls aus einem Silikon mit einer adhäsiven Oberfläche gebildet, wobei ein Pelottenelement 23 auf einem Trägerelement, insbesondere auf einer Trägerfolie aufgebracht ist, so dass auch hier das Pelottenelement 23 selbst die Haftfläche 27 der Pelotte 15 bildet. Die Pelotte 15 ist an der in der Tragstellung zum Träger gerichteten Seite 31 im Wesentlichen eben oder leicht konkav gekrümmt ausgebildet. Die Außenseite der Pelotte 15, also die in der Tragestellung zum Träger abgewandten Seite, ist hierbei bevorzugt konvex gekrümmt.

Die Figur 3B zeigt nun einen Tragezustand des Kompressionsbekleidungssets 3, in dem nur die Fußpelotte 15 an dem Fuß eines Trägers, insbesondere in einem Spannbereich 51, positioniert und dort lösbar befestigt ist. Dies erfolgt durch die Haftfläche 27, die an der in der Tragstellung zum Träger gerichteten Seite 31 an der Pelotte 15 angebracht ist. Wie in den Ausführungsbeispielen zuvor bildet auch hier das Pelottenelement 23 selbst, also dessen Material, insbesondere das verwendete Silikon, die Haftfläche 27 aus. Dadurch ist die Pelotte 15 ohne zusätzliche Hilfe an der Haut des Trägers positionier- und dort lösbar befestigbar.

In Figur 3C ist nun das Kompressionsbekleidungsset 3 in einem Tragezustand gezeigt. Zusätzlich zu der in Figur 3B bereits positionierten und angelegten Fußpelotte 15 ist nun auch das einstellbare Kompressionstextil 9, insbesondere der Compression Wrap, mit dem Grundkörper 47 und den mehreren Bändern 48, 49 an dem Fuß des Trägers, insbesondere in dem Spannbereich 51, angelegt. Dabei erstreckt sich das Kompressionstextil 9 über die Pelotte 15 und kann so Druck auf diese ausüben. Die Kompression, insbesondere die Kompressionsstärke, kann hierbei durch die Bänder 48, 49 individuell eingestellt werden, dies je nach Dehnung der Bänder 48, 49. Je mehr die Bänder 48, 49 gedehnt und anschließend auf sich selbst oder dem Grundkörper 47 mittels der Verschlüsse 50 befestigt werden, desto größer ist der Druck, der auf das Körperteil und auf die Pelotte 15 ausgeübt wird. Durch diese erfindungsgemäße Ausbildung des Kompressionsbekleidungssets 3, wird die An- und Ausziehbarkeit auch von diesem Ausführungsbeispiel des Kompressionsbekleidungssets 3 wesentlich verbessert. Durch das vorherige Positionieren und Befestigen der Pelotte 15 an dem Körperteil, wird eine exakte Positionierung der Pelotte 15 an dem Körperteil sichergestellt. Die Gefahr eines Verrutschens der Pelotte 15 während des Anlegens des Kompressionstextils 9 wird vermieden. Auch kommt es zu keinem Verrutschen der Pelotte 15 bei einem Einstellen der Kompression durch die mehreren Bänder 48, 49, da die Pelotte 15 an dem Körperteil des Trägers befestigt ist und nicht wie im Stand der Technik am Kompressionsartikel.

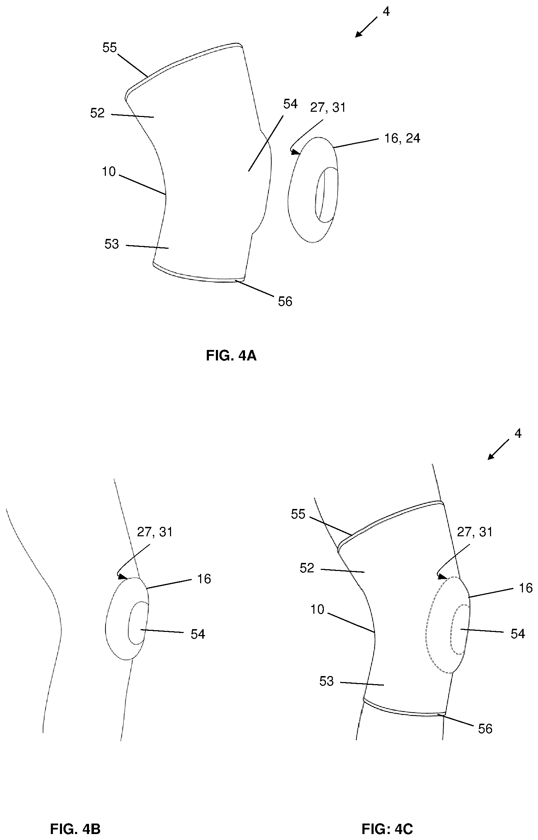

Die Figuren 4A bis 4C zeigen nun ein Kompressionsbekleidungsset 4 mit einem Kompressionsartikel in Form einer Kniebandage 10 sowie einer Kniepelotte 16. Figur 4A zeigt das Set 4 in einem ungetragenen Zustand. In Figur 4B ist die Kniepelotte 16 an dem Bein eines Trägers positioniert. Schließlich zeigt Figur 4C die Bandage 10 und die Kniepelotte 16 in dem getragenen Zustand.

Die in Figur 4A gezeigte kompressive Kniebandage 10 des Kompressionsbekleidungssets 4 besteht im Wesentlichen aus einem Oberschenkelteil 52 sowie aus einem Unterschenkelteil 53. Beide Teile umschließen dabei einen Patellarbereich 54. Am oberen und unteren Ende weist die Bandage 10 ferner jeweils einen Abschlussrand 55 und 56 auf. Die Kniebandage 10 dient zur Stabilisierung des Gelenks. Die Bandage 10 besteht hierbei bevorzugt aus einem textilen, insbesondere gestickten, schlauchförmigen und kompressiven Grundkörper.

Neben der Bandage 10 weist das Kompressionsbekleidungsset 4 die Kniepelotte 16 auf. Diese dient im Wesentlichen dazu Schwellungen im Bereich des Knies und der Patellar abzubauen. Auch diese C- oder O-förmig ausgebildete Kniepelotte 16 weist erfindungsgemäß an der in der Tragstellung zum Träger gerichteten Seite 31 eine Haftfläche 27 auf. Die Kniepelotte 16 ist dadurch losgelöst, also unabhängig, von der Bandage 10 an der Haut und dem Körperteil eines Trägers positionierbar und dort lösbar befestigbar. Wie in den Ausführungsbeispielen zuvor, besteht auch die Pelotte 16 bevorzugt aus einer Trägerfolie, an der ein Pelottenelement 24 angebracht ist. Das Pelottenelement 24 ist auch hier bevorzugt aus einem Silikon mit einer adhäsiven Oberfläche ausgebildet, so dass das Pelottenelement 24 selbst die Haftfläche 27 bildet. Auf der in der Tragstellung zum Träger gerichteten Seite 31 weist das Pelottenelement 24 bevorzugt eine im Wesentlichen ebene bzw. leicht konkav gekrümmte Oberfläche auf. Die Außenseite der Pelotte 16 hingegen ist hierbei konvex gekrümmt ausgebildet.

In Figur 4B ist nun die Kniepelotte 16 an dem Knie des Trägers angebracht. Dazu wird die Pelotte 16, welche an der in der Tragstellung zum Träger gerichteten Seite 31 die Haftfläche 27 aufweist, im Bereich der Patellar 54 positioniert und dort mit leichtem Druck, an dem Körperteil insbesondere an der Haut des Trägers befestigt. Durch die bevorzugt leicht klebrige, adhäsive, jedenfalls selbsthaftende Oberfläche haftet die Pelotte 16 ohne externe Hilfe an der Haut des Trägers.

Figur 4C veranschaulicht nun einen Tragezustand, in dem auch der Kompressionsartikel, nämlich die Kniebandage 10, des Kompressionsbekleidungssets 4 an dem Bein des Trägers angelegt ist. Der Oberschenkelteil 52 mit dem Abschluss 55 der Bandage 10 kommt im Bereich eines Oberschenkels, der Unterschenkelteil 53 mit dem Abschluss 56 im Bereich des Unterschenkels zum Liegen. Die kompressive Bandage 10 übt hierbei jeweils Druck auf das Bein des Trägers aus. Zudem bedeckt die kompressive Bandage 10 die Pelotte 16 und übt ebenso Druck auf diese aus. Hierdurch werden Schwellungen, insbesondere im Bereich des Knies und der Patellar, abgebaut. Dank der Erfindung ist es nun nicht mehr notwendig eine Kniebandage gleichzeitig mit einer eingearbeiteten Pelotte, so wie sie aus dem Stand der Technik bekannt ist, gemeinsam entlang des Beins eines Trägers bis in eine Trageposition, nämlich bis in einen Kniebereich, zu ziehen. Die An- und Ausziehbarkeit des erfindungsgemäßen Kompressionsbekleidungssets 4 wird somit gegenüber den bereits bekannten Lösungen, insbesondere Kniebandagen, deutlich erleichtert.

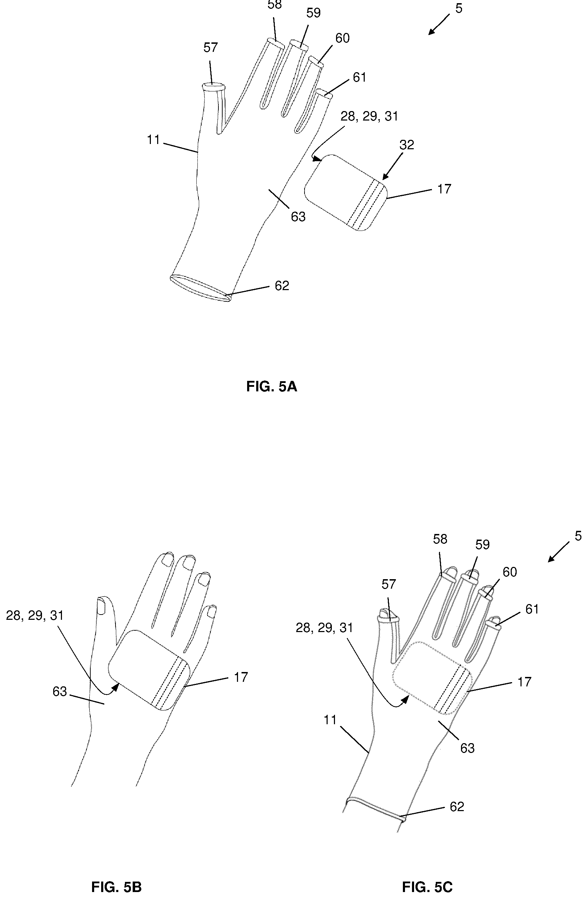

Die Figuren 5A bis 5C zeigen ein weiteres Ausführungsbeispiel des erfindungsgemäßen Kompressionsbekleidungssets 5 mit einem Kompressionsartikel, ausgebildet als kompressiver Handschuh 11, sowie einer Handpelotte 17. In Figur 5A ist das Set 5 erneut in einem ungetragenen Zustand gezeigt. In Figur 5B ist die Handpelotte 17 an der Hand eines Trägers positioniert und dort befestigt. Figur 5C zeigt schließlich den Handschuh 11 und die Handpelotte 17 in dem getragenen Zustand.

Der in Figur 5A gezeigte kompressive Handschuh 11 des Kompressionsbekleidungssets 5 besteht aus einem kompressiven Grundkörper, welcher die Hand, insbesondere die Innenseite der Hand als auch den Handrücken 63, umgibt sowie aus mehreren Fingerabschnitten 57 bis 61. Die bevorzugt ebenfalls kompressiven Fingerabschnitte 57 bis 61 können an ihren Enden, wie gezeigt, offen ausgebildet sein. Denkbar ist es aber auch, dass diese geschlossen sind und dadurch die mehreren Finger eines Trägers vollständig bedecken. Alternativ kann der Handschuh auch ohne Fingerabschnitte ausgebildet sein. An dem gegenüberliegenden Ende des Grundköpers weist dieser einen Abschlussrand 62 oder alternativ einen Bundabschnitt auf. Der kompressive Handschuh 11 dient bevorzugt zur Ödemtherapie im Bereich der Hand. Dabei sorgt der Handschuh 11 insbesondere für einen optimalen Rückfluss der Lymphflüssigkeit aus dem Gewebe der Hand und verhindern so, dass sich diese in den Händen und in den Fingern staut. Dabei besteht der Handschuh 11 bevorzugt aus einem textilen, schlauchförmigen und kompressiven Gestrick.

Die Handpelotte 17, welche ebenfalls Bestandteil des Kompressionsbekleidungssets 5 ist, ist hierbei bevorzugt pad-förmig ausgebildet. Diese dient dazu, Schwellungen im Bereich der Hand, insbesondere im Bereich des Handrückens 63, abzubauen. Auch diese Handpelotte 17 weist erfindungsgemäß nun an der in der Tragstellung zum Träger gerichteten Seite 31 eine Haftfläche 28 auf. Im Gegensatz zu den vorherigen Ausführungsbeispielen ist nun die Haftfläche 28 der Pelotte 17 durch zumindest eine an der Pelotte 17 angebrachten Klebeschicht 29 ausgebildet. Die pad-förmige Pelotte 17 selbst ist hierbei ebenfalls aus einem elastischen Kunststoffmaterial gebildet. Die Handpelotte 17 ist durch die Klebeschicht 29 losgelöst, also unabhängig, von dem Handschuh 11 an der Hand eines Trägers positionierbar und dort lösbar befestigbar. Um nun die Größe der Handpelotte 17 anpassen zu können, weist die Pelotte 17 zusätzlich eine oder mehrere Schneidemarkierungen 32 auf. Hierdurch ist es möglich die Pelotte 17 in ihrer Form und Größe individuell an die Hand, insbesondere an den Handrücken 63, eines Trägers anzupassen. Auf die geometrische Ausbildung der Pelotte wird in den Figuren 8A bis 8C noch näher eingegangen.

In Figur 5B ist nun die Handpelotte 17 an der Hand des Trägers angebracht. Dazu wird die Pelotte 17, welche an der in der Tragstellung zum Träger gerichteten Seite 31 die Haftfläche 28 aufweist, im Bereich des Handrückens 63 positioniert und dort mit leichtem Druck, an der Hand des Trägers befestigt. Durch die bevorzugt leicht klebrige Schicht 29 haftet die Pelotte 17 ohne externe Hilfe an der Hand des Trägers.

Figur 5C zeigt nun einen Tragezustand, in dem auch der Handschuh 11 des Kompressionsbekleidungssets 5 an der Hand des Trägers angelegt ist. Der kompressive Handschuh 11 mit den mehrere Fingerabschnitten 57 bis 61 und dem Abschlussrand 62 übt hierbei Druck auf die Hand, insbesondere auf den Handrücken 63 und somit auch auf die Pelotte 17 aus, welche bereits zuvor durch die an der in der Tragstellung zum Träger gerichteten Seite 31 angeordnete Haftfläche 28, insbesondere Klebeschicht 29, an der Hand des Trägers befestigt wurde. Hierdurch werden Schwellungen, insbesondere im Bereich des Handrückens 63, abgebaut. Wie bereits zuvor erwähnt, ist es auch bei diesem Ausführungsbeispiel nicht notwendig, den Kompressionsartikel 11 samt Pelotte 17 über die Hand eines Trägers zu ziehen, was die An- und Ausziehbarkeit des Kompressionsbekleidungssets 5 deutlich erleichtert.

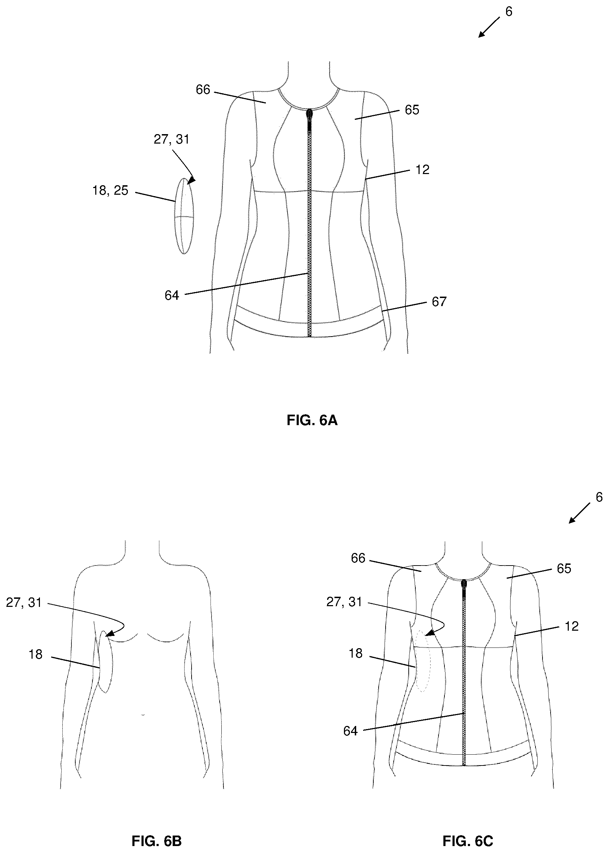

Schließlich zeigen die Figuren 6A bis 6C ein weiteres Ausführungsbeispiel des erfindungsgemäßen Kompressionsbekleidungssets 6. Dabei ist der Kompressionsartikel als kompressive Weste 12 ausgebildet. Die Pelotte ist hierbei als Thoraxpelotte 18 ausgeformt. In Figur 6A ist das Set 6 in einem ungetragenen Zustand gezeigt. In Figur 6B ist die Thoraxpelotte 18 an dem Oberkörper eines Trägers positioniert und dort befestigt. Figur 4C zeigt schließlich die kompressive Weste 12 und die Thoraxpelotte 18 in dem getragenen Zustand.

Wie in Figur 6A gezeigt, ist der Kompressionsartikel des Kompressionsbekleidungssets 6 als kompressive Weste 12 ausgebildet. Die bevorzugt ärmellose Weste 12 besteht hierbei im Wesentlichen aus einem Grundkörper, an welchem bevorzugt zumindest ein Verschluss 64, insbesondere ein Reißverschluss, zum Öffnen und Schließen der Weste 12, angebracht ist. An dem unteren Rand der Weste 12 ist an dem Grundkörper bevorzugt ein Bundabschnitt 67 vorgesehen. Um nun Druck bzw. Kompression auf den Thorax ausüben zu können, weist die Weste 12 zumindest eine, bevorzugt mehrere Kompressionszonen 65 und 66 auf. Diese Zonen sind bevorzugt aus einem weniger elastischen Material gefertigt als der restliche Grundkörper der Weste 12, welcher bevorzugt aus einem im Wesentlichen elastischen Gewebe, bevorzugt Textilgewebe, gefertigt ist. Die Weste 12 kann hierbei bevorzugt mehrschichtig ausgebildet sein, insbesondere um die mehreren Kompressionszonen 65 und 66 auszubilden.

Die Thoraxpelotte 18, welche ebenfalls Bestandteil des Kompressionsbekleidungssets 6 ist und dazu dient Schwellungen im Bereich des Thorax abzubauen, weist hierbei eine ovale Form auf. Auf der in der Tragstellung zum Träger gerichteten Seite 31 weist diese bevorzugt eine im Wesentlichen konkav gekrümmte Oberfläche auf. Die Außenseite der Pelotte 18 hingegen ist hierbei konvex gekrümmt ausgebildet. Auch diese Thoraxpelotte 18 weist erfindungsgemäß an der in der Tragstellung zum Träger gerichteten Seite 31 eine Haftfläche 27 auf, so dass diese losgelöst, also unabhängig, von der Wese 12 an der Haut und dem Thorax eines Trägers positionierbar und dort lösbar befestigbar ist. Wie teilweise in den Ausführungsbeispielen zuvor, besteht auch die Pelotte 18 bevorzugt aus einer Trägerfolie, an der ein Pelottenelement 25 angebracht ist. Das Pelottenelement 25 ist auch hier bevorzugt aus einem Silikon mit einer adhäsiven Oberfläche ausgebildet, so dass das Pelottenelement 25 selbst die Haftfläche 27 bildet.

In Figur 6B ist nun die Thoraxpelotte 18 an dem Oberkörper des Trägers angebracht. Dies erfolgt dadurch, dass die Pelotte 18, welche an der in der Tragstellung zum Träger gerichteten Seite 31 die Haftfläche 27 aufweist, im Bereich des Oberkörpers positioniert und dort mit leichtem Druck befestigt wird. Durch die bevorzugt leicht klebrige Schicht 27 haftet die Pelotte 18 an der haut des Oberkörpers.

Figur 6C zeigt nun den Tragezustand, in dem auch die Weste 12 des Kompressionsbekleidungssets 6 an dem Thorax des Trägers angelegt ist. Die mittels des Verschlusses 64 geschlossene Weste 12, mit den mehreren Kompressionszonen 65 und 66, übt hierbei Druck auf den Thorax und auf die Pelotte 18 aus, welche mittels der Haftfläche 27 auf der Seite 31 an dem Thorax positioniert ist. Hierdurch werden Schwellungen im Thorax abgebaut.

Die Figuren 7A bis 7C zeigen nun die Knöchelpelotte 13 des ersten Ausführungsbeispiels des erfindungsgemäßen Kompressionsbekleidungssets 1, insbesondere aus den Figuren 1A bis 1C, im Detail.

In Figur 7A ist die L-förmige Pelotte 13 in der Draufsicht gezeigt, also von der dem Träger abgewandten Seite 30. Auf dieser Außenseite 30 ist die Pelotte 13 konvex gekrümmt. Diese Außenseite 30 wird hierbei durch das Trägerelement 19 der Pelotte 13, insbesondere durch die Trägerfolie, gebildet, an welcher das Pelottenelement 21 angeordnet ist. Die Trägerfolie 19 weist hierbei eine friktionsarme Oberfläche auf, so dass die Pelotte 13 an der dem Kompressionsartikel 7 zugewandten Seite erhöhte Gleiteigenschaften aufweist. Das Anziehen des Kompressionsartikels, insbesondere des Beinstrumpfes 7, wird durch die friktionsarme Oberfläche an der Außenseite 30 der Pelotte 13 deutlich erleichtert.

Figur 7B zeigt nun die Knöchelpelotte 13 von unten, insbesondere von der dem Träger zugewandten Seite 31. Die Pelotte 13 weist an dieser einer in der Tragstellung zum Träger gerichteten Seite 31 die Haftfläche 27 auf, um die Pelotte 13 losgelöst von dem Kompressionsartikel 7 an der Haut eines Trägers positionieren zu können. Die Haftfläche 27 ist hierbei durch die adhäsive Oberfläche 26 des Pelottenelements 21 gebildet. Die Seite 31 ist hierbei durch eine ebene Oberfläche ausgebildet.

In Figur 7C ist nun die Knöchelpelotte 13 in einer Schnittdarstellung entlang der Linie AA gemäß der Figur 7A gezeigt. Die Außenseite 20 wird durch die Trägerfolie 19 gebildet, an der das Pelottenelement 21 angebracht ist. Dieses besteht, wie bereits zuvor erwähnt, aus einem elastischen Kunststoffmaterial, insbesondere aus einem Silikon, so dass das Pelottenelement 21 selbst mit der adhäsiven Oberfläche 26 die zumindest eine Haftfläche 27 auf der dem Träger zugewandten Seite 31 bildet. Die in der Tragestellung zum Träger abgewandten Seite 30 ist stark gekrümmt, insbesondere konvex, ausgebildet. An der Haftfläche 27 ist nun ein Abdeckelement 33 lösbar anbringbar. Dadurch ist die Haftfläche 27 der Pelotte 13 in einem freiliegenden Zustand vor Verunreinigungen geschützt. Das lösbare Abdeckelement 33 ist dabei bevorzugt als Kunststofffolie ausgebildet, welche die gesamte Haftfläche 27 bedeckt.

Nicht dargestellt, aber erfindungsgemäß bevorzugt auch Bestandteil des Kompressionsbekleidungssets 1, ist ein Mittel zur Reinigung der Haftfläche 27, so dass die zumindest eine Pelotte 13 wiederverwendbar ist. Das Mittel ist dabei bevorzugt derart ausgebildet, dass es Öl- und Fettverschmutzungen, Staub, Schmutz, Make-Up, Hautschuppen, Hautpartikel und Haare von der Haftschicht 27 löst, ohne die Haftschicht 27 dabei zu beschädigen.

Die Figuren 8A bis 8C zeigen schließlich die Handpelotte 17 des fünften Ausführungsbeispiels des erfindungsgemäßen Kompressionsbekleidungssets 5, insbesondere aus den Figuren 5A bis 5C, im Detail.

In Figur 8A ist die pad-förmige Pelotte 17 in der Draufsicht gezeigt, also von der dem Träger abgewandten Seite 30. Wie zu sehen ist, weist die Pelotte 17 an dieser Seite 30 eine oder mehrere Schneidemarkierungen 32 auf, um die Größe der Handpelotte 17 individuell an die Größe eines Handrückens anpassen zu können. Die pad-förmige Pelotte 17 selbst ist hierbei ebenfalls aus einem Kunststoffmaterial gebildet.

Figur 8B zeigt nun die Unterseite der Pelotte 17, nämlich die einem Träger zugewandte Seite 31. An dieser Seite 31 weist die Handpelotte 17 die Haftfläche 28 auf. Gemäß diesem Ausführungsbeispiel ist nun die Haftfläche 28 der Pelotte 17 durch zumindest eine an der Pelotte 17 angebrachten Klebeschicht 29 ausgebildet, wodurch die Pelotte 17 losgelöst, also unabhängig, von einem Handschuh an der Hand eines Trägers positionierbar und dort lösbar befestigbar ist. Um nun Schwellungen im Bereich der Hand, insbesondere im Bereich des Handrückens, abzubauen, weist die Pelotte 17 an der Seite 31 mehrere Rücksprung 34 auf. Die Rücksprünge 34 dienen dazu, in der Tragestellung unterhalb der Pelotte 17 einen, bevorzugt mehrere, Transportkanäle 34, insbesondere für den Abtransport der Lymphflüssigkeit auszubilden.

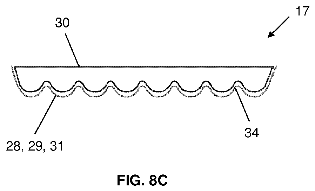

In Figur 8C ist nun die Handpelotte 17 in einer Schnittdarstellung entlang der Linie BB gemäß der Figur 8A gezeigt. Wie zu sehen ist, ist die in der Tragestellung zum Träger abgewandten Seite 30 im Wesentlichen eben ausgebildet. An der gegenüberliegenden Seite 31, mit der Haftfläche 28 in Form einer zusätzlichen Klebeschicht 29, welche an der Pelotte 17 angebracht bzw. aufgebracht ist, weist die Pelotte 17 die mehreren, insbesondere in diesem Ausführungsbeispiel sieben, Rücksprünge 34 auf. Die Rücksprünge 34 und die Innenseite 31 der Pelotte 17 bildet hierbei eine bevorzugt wellenförmige Oberfläche bzw. Haftfläche 28 mit mehreren Transportkanälen 34 für Lymphflüssigkeit aus. Alternativ sind natürlich auch andere geometrische Ausbildungen der Rücksprünge 34 denkbar. Bspw. können diese v- oder u-förmige ausgebildet sein. Alternativ können auch beabstandete Pelottenelemente vorgesehen sein. Die Klebeschicht 29 kann auch aus mehreren Abschnitten bestehen.

Die Erfindung ist nicht auf die beschriebenen Ausführungsbeispiele beschränkt, sondern umfasst alle Ausführungen, die das prinzipielle, sinngemäße Funktionsprinzip der Erfindung anwenden oder beinhalten. Des Weiteren sind alle Merkmale aller beschriebenen und dargestellten Ausführungsbeispiele miteinander kombinierbar.

## Patentansprüche

1. Kompressionsbekleidungsset (1, 2, 3, 4, 5, 6) zur Ausübung einer Kompression auf ein Körperteil bestehend aus zumindest einem Kompressionsartikel (7, 8, 9, 10, 11, 12) sowie zumindest einer dem Kompressionsartikel (7, 8, 9, 10, 11, 12) zugehörigen Pelotte (13, 14, 15, 16, 17, 18), wobei die Pelotte (13, 14, 15, 16, 17, 18) in der Tragestellung des Kompressionsbekleidungssets (1, 2, 3, 4, 5, 6) unter dem Kompressionsartikel (7, 8, 9, 10, 11, 12) zum Liegen kommt, wobei die Pelotte (13, 14, 15, 16, 17, 18) an einer in der Tragstellung zum Träger gerichteten Seite (31) zumindest eine Haftfläche (27, 28) aufweist, so dass die Pelotte (13, 14, 15, 16, 17, 18) losgelöst von dem Kompressionsartikel (7, 8, 9, 10, 11, 12) an der Haut eines Trägers positionierbar ist, **dadurch gekennzeichnet, dass** die zumindest eine Pelotte (13, 14, 15, 16, 18) an einer in der Tragestellung zum Träger abgewandten Seite (30) zumindest ein Trägerelement (19) für zumindest ein daran angebrachtes Pelottenelement (21, 22, 23, 24, 25) aufweist, wobei das zumindest eine Pelottenelement (21, 22, 23, 24, 25) aus einem Silikon mit einer adhäsiven Oberfläche (26) besteht, so dass das Pelottenelement (21, 22, 23, 24, 25) selbst die zumindest eine Haftfläche (27) bildet oder die zumindest eine Haftfläche (28) durch eine an der Pelotte (17) oder dem zumindest einen Pelottenelement aufgebrachte Klebeschicht (29) gebildet ist.

2. Kompressionsbekleidungsset (1, 2, 3, 4, 6) nach Anspruch 1, **dadurch gekennzeichnet, dass** das zumindest eine Trägerelement (19) aus einer Trägerfolie mit einer friktionsarmen Oberfläche an der Außenseite (20) gefertigt ist, so dass die Pelotte (13, 14, 15, 16, 18) an der dem Kompressionsartikel (7, 8, 9, 10, 12) zugewandten Seite (30) erhöhte Gleiteigenschaften aufweist.

3. Kompressionsbekleidungsset (1, 2, 3, 4, 5, 6) nach einem der vorherigen Ansprüche, **dadurch gekennzeichnet, dass** die zumindest eine Pelotte (17) aus einem elastischen oder plastisch verformbaren Kunststoffmaterial besteht.

4. Kompressionsbekleidungsset (1, 2, 3, 4, 5, 6) nach einem der vorherigen Ansprüche, **dadurch gekennzeichnet, dass** die Pelotte (17) oder das zumindest eine Pelottenelement (21, 22, 23, 24, 25) auf der in der Tragestellung zum Träger gerichteten Seite (31) im Wesentlichen eben, konvex oder konkav gekrümmt, ausgebildet ist.

5. Kompressionsbekleidungsset (5) nach einem der vorherigen Ansprüche, **dadurch gekennzeichnet, dass** die Pelotte (17) oder das zumindest eine Pelottenelement auf der in der Tragstellung zum Träger gerichteten Seite (31) zumindest einen Rücksprung (34) aufweist oder an dem Trägerelement mehrere aneinander anschließende oder voneinander beabstandete Pelottenelemente angebracht sind.

6. Kompressionsbekleidungsset (1, 2, 3, 4, 6) nach einem der vorherigen Ansprüche **dadurch gekennzeichnet, dass** die zumindest eine Pelotte (13, 14, 15, 16, 18) an einer in der Tragestellung zum Träger abgewandten Seite (30) im Wesentlichen konvex gekrümmt ist.

7. Kompressionsbekleidungsset (5) nach einem der vorherigen Ansprüche, **dadurch gekennzeichnet, dass** die Pelotte (17) eine oder mehrere Schneidemarkierungen (32) aufweist, so dass die Pelotte (17) in ihrer Form individuell anpassbar ist.

8. Kompressionsbekleidungsset (1) nach einem der vorherigen Ansprüche, **dadurch gekennzeichnet, dass** das Kompressionsbekleidungsset (1) ferner zumindest ein lösbar an der zumindest einen Haftfläche (27) der Pelotte (13) befestigbares Abdeckelement (33) aufweist, so dass die Haftfläche (27) der Pelotte (13) in einem freiliegenden Zustand vor Verunreinigungen geschützt ist.

9. Kompressionsbekleidungsset (1, 2, 3, 4, 5, 6) nach einem der vorherigen Ansprüche, **dadurch gekennzeichnet, dass** das Set (1, 2, 3, 4, 5, 6) ferner ein Mittel zur Reinigung der Haftfläche (27, 28) umfasst, so dass die zumindest eine Pelotte (13, 14, 15, 16, 17, 18) wiederverwendbar ist.

10. Kompressionsbekleidungsset (1, 2, 3, 4, 5, 6) nach einem der vorherigen Ansprüche, **dadurch gekennzeichnet, dass** der Kompressionsartikel ein Strumpf, insbesondere ein Arm- oder Beinstumpf (7), eine Socke, eine Zehenkappe (8), ein Handschuh (9), eine Bandage (10), ein einstellbares Kompressionstextil (11), eine Weste (12), oder ein kompressives Element einer Orthese ist.

11. Kompressionsbekleidungsset (1, 2, 3, 4, 5, 6) nach einem der vorherigen Ansprüche, **dadurch gekennzeichnet, dass** die durch den Kompressionsartikel (7, 8, 9, 10, 11, 12) erzeugten kompressiven Druckwerte zwischen 5 und 60 mmHg, bevorzugt zwischen 10 und 45 mmHg, betragen.

## Claims

1. Compression garment set (1, 2, 3, 4, 5, 6) for applying compression to a body part consisting of at least one compression article (7, 8, 9, 10, 11, 12) as well as at least one pressure pad (13, 14, 15, 16, 17, 18) pertaining to the compression article (7, 8, 9, 10, 11, 12), wherein in the wearing position of the compression garment set (1, 2, 3, 4, 5, 6) the pressure pad (13, 14, 15, 16, 17, 18) comes to lie under the compression article (7, 8, 9, 10, 11, 12) wherein the pressure pad (13, 14, 15, 16, 17, 18) has at least one adhesive surface (27, 28) on a side (31) directed towards the wearer in the wearing position so that the pressure pad (13, 14, 15, 16, 17, 18) can be positioned on the skin of a wearer detached from the compression article (7, 8, 9, 10, 11, 12), **characterized in that** the at least one pressure pad (13, 14, 15, 16, 18) has at least one support element (19) for at least one pressure pad element (21, 22, 23, 24, 25) attached thereto on a side (30) facing away from the wearer in the wearing position, wherein the at least one pressure pad element (21, 22, 23, 24, 25) consists of a silicone with an adhesive surface (26) so that the pressure pad element (21, 22, 23, 24, 25) itself forms at least one adhesive surface (27) or the at least one adhesive surface (28) is formed by an adhesive layer (29) attached to the pressure pad (17) or to the at least one pressure pad element.

2. Compression garment set (1, 2, 3, 4, 6) according to Claim 1, **characterized in that** the at least one support element (19) is fabricated from a support film having a low-friction surface on the outer side (20) so that the pressure pad (13, 14, 15, 16, 18) has enhanced sliding properties on the side (30) facing the compression article (7, 8, 9, 10, 12).

3. Compression garment set (1, 2, 3, 4, 5, 6) according to one of the preceding claims, **characterized in that** the at least one pressure pad (17) consists of an elastic or plastically deformable plastic material.

4. Compression garment set (1, 2, 3, 4, 5, 6) according one of the preceding claims, **characterized in that** the pressure pad (17) or the at least one pressure pad element (21, 22, 23, 24, 25) is formed on the side (31) directed towards the wearer in the wearing position, substantially flat, convex or concavely curved.

5. Compression garment set (5) according to one of the preceding claims, **characterized in that** the pressure pad (17) or the at least one pressure pad element has at least one recess (34) on the side (31) directed towards the wearer in the wearing position or a plurality of adjoining or spaced-apart pressure pad elements are attached to the support element.

6. Compression garment set (1, 2, 3, 4, 6) according to one of the preceding claims, **characterized in that** the at least one pressure pad (13, 14, 15, 16, 18) is substantially convexly curved on a side (30) facing away from the wearer in the wearing position.

7. Compression garment set (5) according to one of the preceding claims, **characterized in that** the pressure pad (17) has one or more cutting markings (32) so that the pressure pad (17) can be individually adapted in its shape.

8. Compression garment set (1) according to one of the preceding claims, **characterized in that** the compression garment set (1) further comprises at least one cover element (33) that can be detachably attached to the at least one adhesive surface (27) of the pressure pad (13) so that the adhesive surface (27) of the pressure pad (13) in an exposed state is protected from contaminants.

9. Compression garment set (1, 2, 3, 4, 5, 6) according to one of the preceding claims, **characterized in that** the set (1, 2, 3, 4, 5, 6) further comprises a means for cleaning the adhesive surface (27, 28) so that the at least one pressure pad (13, 14, 15, 16, 17, 18) can be reused.

10. Compression garment set (1, 2, 3, 4, 5, 6) according to one of the preceding claims, **characterized in that** the compression article is a stocking, in particular an arm or leg stocking (7), a sock, a toe cap (8), a glove (9), a bandage (10), an adjustable compression textile (11), a waistcoat (12) or a compressive element of an orthesis.

11. Compression garment set (1, 2, 3, 4, 5, 6) according to one of the preceding claims, **characterized in that** the compressive pressures generated by the compression article (7, 8, 9, 10, 11, 12) are between 5 and 60 mm Hg, preferably between 10 and 45 mm Hg.

## Revendications

1. Kit de vêtement de compression (1, 2, 3, 4, 5, 6), destiné à exercer une compression sur une partie du corps, constitué d'au moins un article de compression (7, 8, 9, 10, 11, 12), ainsi que d'au moins un insert (13, 14, 15, 16, 17, 18), appartenant à l'article de compression (7, 8, 9, 10, 11, 12), dans la position portée du kit de vêtement de compression (1, 2, 3, 4, 5, 6), l'insert (13, 14, 15, 16, 17, 18) venant se situer sous l'article de compression (7, 8, 9, 10, 11, 12), sur un côté (31) dirigé vers le porteur dans la position portée, l'insert (13, 14, 15, 16, 17, 18) comportant au moins une surface adhérente (27, 28), de sorte que l'insert (13, 14, 15, 16, 17, 18) soit positionnable sur la peau d'un porteur en étant désolidarisé de l'article de compression (7, 8, 9, 10, 11, 12), **caractérisé en ce que** sur un côté (30) opposé au porteur dans la position portée, l'au moins un insert (13, 14, 15, 16, 18) comporte au moins un élément de support (19) pour au moins un élément d'insert (21, 22, 23, 24, 25), monté sur celui-ci, l'au moins un élément d'insert (21, 22, 23, 24, 25) étant constitué d'un silicone pourvu d'une surface (26) adhésive, de sorte que l'élément d'insert (21, 22, 23, 24, 25) constitue lui-même l'au moins une surface adhérente (27) ou que l'au moins une surface adhérente (28) soit constituée par une couche adhésive (29) appliquée sur l'insert (17) ou sur l'au moins un élément d'insert.

2. Kit de vêtement de compression (1, 2, 3, 4, 6) selon la revendication 1, **caractérisé en ce que** l'au moins un élément de support (19) est fabriqué en une feuille de support dotée d'une surface à faible friction sur la face extérieure (20), de sorte que, sur le côté (30) dirigé vers l'article de compression (7, 8, 9, 10, 12) l'insert (13, 14, 15, 16, 18) fasse preuve de caractéristiques de glissement élevées.

3. Kit de vêtement de compression (1, 2, 3, 4, 5, 6) selon l'une quelconque des revendications précédentes, **caractérisé en ce que** l'au moins un insert (17) est constitué d'une matière plastique élastiquement ou plastiquement déformable.

4. Kit de vêtement de compression (1, 2, 3, 4, 5, 6) selon l'une quelconque des revendications précédentes, **caractérisé en ce que** sur le côté (31) dirigé vers le porteur dans la position portée, l'insert (17) ou l'au moins un élément d'insert (21, 22, 23, 24, 25) est conçu de manière sensiblement plane, convexe ou incurvée de forme concave.

5. Kit de vêtement de compression (5) selon l'une quelconque des revendications précédentes, **caractérisé en ce que** sur le côté (31) dirigé vers le porteur dans la position portée, l'insert (17) ou l'au moins un élément d'insert comporte au moins un renfoncement (34) ou **en ce que** sur l'élément de support sont montés plusieurs éléments d'insert se raccordant les uns sur les autres, ou écartés les uns des autres.

6. Kit de vêtement de compression (1, 2, 3, 4, 6) selon l'une quelconque des revendications précédentes **caractérisé en ce que** sur un côté (30) opposé au porteur dans la position portée, l'au moins un insert (13, 14, 15, 16, 18) est incurvé de manière sensiblement convexe.

7. Kit de vêtement de compression (5) selon l'une quelconque des revendications précédentes, **caractérisé en ce que** l'insert (17) comporte une ou plusieurs marques de coupe (32), de sorte que l'insert (17) soit individuellement adaptable dans sa forme.

8. Kit de vêtement de compression (1) selon l'une quelconque des revendications précédentes, **caractérisé en ce que** le kit de vêtement de compression (1) comporte par ailleurs au moins un élément de recouvrement (33), susceptible d'être fixé de manière amovible sur l'au moins une surface adhérente (27) de l'insert (13), de sorte que dans un état exposé, la surface adhérente (27) de l'insert (13) soit protégée des salissures.

9. Kit de vêtement de compression (1, 2, 3, 4, 5, 6) selon l'une quelconque des revendications précédentes, **caractérisé en ce que** le kit (1, 2, 3, 4, 5, 6) comprend par ailleurs un moyen de nettoyage de la surface adhérente (27, 28), de sorte que l'au moins un insert (13, 14, 15, 16, 17, 18) soit réutilisable.

10. Kit de vêtement de compression (1, 2, 3, 4, 5, 6) selon l'une quelconque des revendications précédentes, **caractérisé en ce que** l'article de compression est un bas, notamment un manchon ou une chaussette (7), une socquette, un embout protecteur d'orteil (8), un gant (9), un bandage (10), un textile de compression (11) réglable, un gilet (12), ou un élément compressif d'une orthèse.

11. Kit de vêtement de compression (1, 2, 3, 4, 5, 6) selon l'une quelconque des revendications précédentes, **caractérisé en ce que** les valeurs de pression compressive générées par l'article de compression (7, 8, 9, 10, 11, 12) se situent entre 5 et 60 mmHg, de préférence entre 10 et 45 mmHg.
